# EUROPEAN PATENT APPLICATION

(11) **EP 4 197 456 A1**
(43) Date of publication of application: **21.06.2023**
(21) Application number: 21860183.9
(22) Date of filing: 13.08.2021
(51) Int. Cl.: A61B 17/12

(54) **HEMANGIOMA BLOCKING DEVICE, HEMANGIOMA BLOCKING TREATMENT DEVICE AND HEMANGIOMA BLOCKING SYSTEM**

(30) Priority: 31.08.2020 CN 202010899196
(71) Applicant: MicroPort NeuroTech (Shanghai) Co., Ltd., Shanghai 201318 (CN)
(72) Inventor: GUO, Shuang, Shanghai 201318 (CN); CHANG, Mengqi, Shanghai 201318 (CN); CHEN, Bing, Shanghai 201318 (CN); GUO, Yuanyi, Shanghai 201318 (CN); PAN, Guangliang, Shanghai 201318 (CN); LU, Huina, Shanghai 201318 (CN)
(74) Representative: Marks & Clerk LLP
(86) International application number: PCT/CN2021/112627
(87) International publication number: WO 2022/042345

(57) **Abstract**

An aneurysm occlusion device (10), a therapeutic apparatus for aneurysm occlusion and an aneurysm occlusion system. The aneurysm occlusion device (10) includes an expandable mesh structure (11) and a guide structure (12). The expandable mesh structure (11) has an expanded configuration, in which it assumes a spiral shape spiraling from a distal end (111) to a proximal end (112) thereof, and a collapsed configuration for its delivery into an aneurysm (30) from a blood vessel. At least a portion of the guide structure (12) is disposed in an inner cavity defined by the expandable mesh structure (11) and, when the expandable mesh structure (11) is in the spiral shape, expands into a spiral shape in the inner cavity of the expandable mesh structure (11). The aneurysm occlusion device (10) provides the advantages of, among others, stably and compliantly packing the aneurysm (30) while avoiding its rupture, preventing vascular embolism, improving coverage of an opening at the neck of the aneurysm, promoting thrombosis in the aneurysm (30) and accelerating embolization of the aneurysm (30).

## Description

### TECHNICAL FIELD

The present invention relates to the technical field of medical instruments and, in particular, to an aneurysm occlusion device, a therapeutic apparatus for aneurysm occlusion and an aneurysm occlusion system.

### BACKGROUND

Intracranial aneurysms are pathological bulges in intracranial artery walls, with an incidence rate of 5% to 10%. A magnetic resonance angiography (MRA) study shows that the morbidity of unruptured aneurysms for Chinese adults aged 35-75 years is approximately 7.0%. Although subarachnoid hemorrhages caused by intracranial aneurysm ruptures account for about 5% of strokes, primary ruptures are associated with a mortality rate of 20-30%, and re-ruptures are associated with a mortality rate of as high as 60%. Aneurysm treatment relies on complete isolation of an aneurysm from blood circulation by a therapeutic method. Currently available therapeutic methods primarily include craniotomy and clipping and endovascular intervention. At present, endovascular intervention has been predominant because it allows direct access to a lesion without affecting the brain tissue and because of its minimally invasive nature. Currently available endovascular interventional therapies are principally as follows:
(1) At present, aneurysm embolization with coils is mostly used in aneurysm treatment. It can achieve aneurysm occlusion and treatment by promoting thrombosis through altering local hemodynamic factors. However, as aneurysms are diverse in shape and size, incomplete coil packing may lead to recanalization of an aneurysm, while excessive packing may cause intraoperative rupture of an aneurysm. This places stringent requirements on physicians' dexterity and experience. Moreover, coil packing may require multiple procedures and therefore lacks efficiency. In some cases, additional use of stents, balloons, and catheters may be required, leading to increased operational complexity. Further, for wide-neck aneurysms, coils tend to herniate into the parent arteries, affecting blood flow, or even causing vascular stenosis in severe cases.
(2) As a significant breakthrough in the field of endovascular treatment of intracranial aneurysms, flow diverters provide a novel approach for the treatment of complex aneurysms. This approach involves placing a dense mesh stent in a parent artery, which enables endoluminal reconstruction of the diseased blood vessel and subsequent luminal surface re-endothelialization by neointimal growth over the aneurysm neck. The use of flow diverters has significantly improved long-term therapeutic benefit for large and giant aneurysms and considerably reduced the use of coils. In addition, according to computer-aided hemodynamic simulation analysis, a metal coverage percentage of 30-50% can significantly reduce intra-aneurysmal blood flow and achieve a high cure rate. However, the use of flow diverters can lead to long-term reliance of patients on dual antiplatelet therapy and a risk of hemorrhagic complications after surgery. Further, some large aneurysms are associated with a risk of rupture a certain period of time after the treatment.
(3) Currently, there are also novel devices capable of embolization in one pass, which are generally made of shape memory materials and shaped into balls, cylinders or discs. Such a device is designed to be delivered through a catheter to a target site, where it is pushed out of a sheath and then self-expand to recover its spherical shape, thereby blocking the aneurysm. For example, a first embolization device is a spherical or cylindrical dense mesh device with anchors at its opposing ends. After full expansion of the device within an aneurysm, a proximal dense mesh thereof covers the neck of the aneurysm to therapeutically treat the aneurysm. A second embolization device is a three-dimensional mesh-like structure composed of a radiopaque wire and surrounding self-expanding shape memory alloy wires. Similar to a coil, this device can be released and retrieved by a catheter and packed within an aneurysm in a spherical form capable of disturbing blood flow. A third embolization device is a braided double-layer nickel-titanium alloy device. A fourth embolization device is a braided double-layer shape memory alloy device, which is in the form of a coiled disc when not stressed. However, when released into an aneurysm, it will be stressed by walls of the aneurysm and assume a tulip-like shape, which allows it to be stabilized in a lower part of the aneurysm while covering the aneurysm neck to provide an effect of hemodynamic remodeling. However, in the design of the first embolization device, the proximal anchor makes the device symmetric and necessary to cover the aneurysm neck in a certain orientation. Therefore, it is used mainly for the treatment of bifurcated wide-neck aneurysms and is particularly suitable for those with a regular morphology. In the design of the first embolization device, the distal anchor may impact an aneurysm wall and is likely to cause a rupture or bleeding of the aneurysm. In some cases, the proximal anchor of the first embolization device may be pushed by the aneurysm wall to herniate into the parent artery, affecting the endothelialization of the aneurysm neck. In addition, the first embolization device is generally in the form of a single ball or cylinder, which is insufficiently strong despite of a large contact area. This is unfavorable to long-term stability, and tends to lead to displacement, of the device within the aneurysm. The three-dimensional mesh-like structure of the second embolization device is spherical and composed of multiple meshed sheets. Due to significant friction between different parts of the three-dimensional mesh-like structure and between the structure itself and aneurysm walls, it is difficult for the device to recover the intended shape and, and even if this is achieved, the device cannot maintain the shape stably for a sufficiently long period of time. Therefore, it cannot provide desirable packing quality. Moreover, since this device must be used in combination with a coil, its operation is complicated. The third embolization device operates in a similar manner as the first embolization device and therefore suffers from similar problems. The fourth embolization device also has a proximal anchor, which likewise tends to be pushed by an aneurysm wall to herniate into the parent artery and makes the device only suitable for use in apex aneurysms. Moreover, as this device requires repeated relocation before it can satisfactorily stay stably within the aneurysm, it lacks efficiency.

### SUMMARY OF THE INVENTION

In order to overcome the above problems, it is an object of the present invention to provide an aneurysm occlusion device, a therapeutic apparatus for aneurysm occlusion and an aneurysm occlusion system, which are used for treatment of aneurysm occlusion, capable of stable and compliant packing and advantageous in, among others, preventing aneurysm rupture and vascular embolism, enabling improved coverage of an aneurysm neck opening, promoting thrombosis in an aneurysm and aneurysm embolization.

To achieve the above object, the present invention provides an aneurysm occlusion device provided in the present invention, which includes:
an expandable mesh structure having an expanded configuration, in which the expandable mesh structure forms a spiral shape by spiraling from a distal end to a proximal end thereof, and a collapsed configuration for delivery into an aneurysm from a blood vessel; and
a guide structure, at least a portion of which is disposed in an inner cavity defined by the expandable mesh structure and, when the expandable mesh structure is in the spiral shape, expands into a spiral shape in the inner cavity of the expandable mesh structure.

Optionally, the guide structure may be at least partially made of a radiopaque material.

Optionally, the spiral shape of the expandable mesh structure in the expanded configuration thereof may be a three-dimensional spiral shape formed by spiraling from the distal end to the proximal end.

Optionally, the guide structure may have a first section that is disposed in the inner cavity of the expandable mesh structure and a second section that extends out of the expandable mesh structure from the distal end thereof.

Optionally, the second section of the guide structure that extends out of the expandable mesh structure from the distal end thereof may have a spiral shape when in the expanded configuration.

Optionally, the second section of the guide structure that extends out of the expandable mesh structure from the distal end thereof may have a three-dimensional spiral shape.

Optionally, the guide structure may have an elongated structure.

Optionally, both the proximal and distal ends of the expandable mesh structure may be fixed connected to the guide structure, and an axial length of a first section of the guide structure that is disposed in the inner cavity of the expandable mesh structure in the expanded configuration may be not smaller than an axial length of the expandable mesh structure in the collapsed configuration.

Optionally, a distal radiopaque ring may be fixedly connected to the distal end of the expandable mesh structure, and/or a proximal radiopaque ring may be fixedly connected to the proximal end of the expandable mesh structure.

Optionally, a cross-sectional area of the expandable mesh structure may increase and then decrease from the proximal end to the distal end.

Optionally, the expandable mesh structure may comprise a proximal section, a middle section and a distal section which are connected axially in sequence, wherein a cross-sectional area of the proximal section gradually increases from a proximal end to a distal end thereof, and/or a cross-sectional area of the distal section gradually increases from a distal end to a proximal end thereof.

Optionally, the cross-sectional area of the expandable mesh structure may repeatedly increase and decrease from the proximal end to the distal end.

Optionally, in the expanded configuration, a maximum outer diameter of the expandable mesh structure may be not smaller than 1/4 of an outer diameter of a largest spiral turn in the expandable mesh structure.

Optionally, in the expanded configuration, the maximum outer diameter of the expandable mesh structure may be 1/3 to 1/2 of the outer diameter of the largest spiral turn in the expandable mesh structure.

Optionally, in the expanded configuration, the maximum outer diameter of the expandable mesh structure may be 2.0-8.0 mm, and the outer diameter of the largest spiral turn in the expandable mesh structure may be 3.0-25 mm.

Optionally, the expandable mesh structure may be formed by braiding filaments made of a material comprising a shape memory material.

Optionally, the expandable mesh structure is formed by braiding 48-144 filaments having an outer diameter of 0.0005-0.002 inches.

Optionally, the expandable mesh structure may be formed by braiding radiopaque filaments, or the expandable mesh structure may be formed by braiding both radiopaque and non-radiopaque filaments.

Optionally, the spiral shape of the expandable mesh structure in the expanded configuration may have one spiral turn or a plurality of spiral turns, wherein
in case of the plurality of spiral turns, a first one of the spiral turns in the expandable mesh structure proximal to the distal end thereof has an outer diameter smaller than an outer diameter of each middle one of the spiral turns.

Optionally, in case of the plurality of spiral turns, a last one of the spiral turns in the expandable mesh structure proximal to the proximal end thereof may have an outer diameter smaller than the outer diameter of each middle spiral turn.

Optionally, in the spiral shape of the expandable mesh structure in the expanded configuration, the outer diameter of the first spiral turn proximal to the distal end may be equal to the outer diameter of the last spiral turn proximal to the proximal end.

Optionally, in the spiral shape of the expandable mesh structure in the expanded configuration, the outer diameter of the first spiral turn proximal to the distal end may be 2/3 of the outer diameter of each middle spiral turn.

Optionally, in case of the expandable mesh structure having a plurality of middle spiral turns, these middle spiral turn may have equal outer diameters.

Optionally, the guide structure may have no less than 1/4 spiral turns at the distal end thereof.

Optionally, the guide structure may have 1/4-3 spiral turns at the distal end thereof.

Optionally, the spiral shape of the second section of the guide structure that extends out of the expandable mesh structure from the distal end thereof in the expanded configuration may have one spiral turn or a plurality of spiral turns, wherein
in case of the plurality of spiral turns, a first one of the spiral turns in the guide structure proximal to the distal end thereof has an outer diameter smaller than an outer diameter of each spiral turn other than the first spiral turn.

Optionally, in case of the spiral shape of the second section of the guide structure that extends out of the expandable mesh structure from the distal end thereof in the expanded configuration having the plurality of spiral turns, the outer diameter of each spiral turn in the guide structure does not exceed an outer diameter of a first spiral turn in the expandable mesh structure proximal to the distal end thereof.

Optionally, the outer diameter of the first spiral turn in the guide structure proximal to the distal end thereof may be 2/3-3/4 of the outer diameter(s) of the other spiral turn(s), which may be equal and not smaller than 2/3 of an outer diameter of a first spiral turn in the expandable mesh structure proximal to the distal end thereof.

To achieve the above object, the present invention provides a therapeutic apparatus for aneurysm occlusion, which includes the aneurysm occlusion device as defined above and a push pod connected to the proximal end of the expandable mesh structure in the aneurysm occlusion device.

Optionally, the push pod may extend in a direction tangential to a spiral contour of the spiral shape of the expandable mesh structure in the expanded configuration.

To achieve the above object, the present invention provides an aneurysm occlusion system, which includes the aneurysm occlusion device as defined above and a catheter, wherein the expandable mesh structure is crimped in the catheter and, after being released from the catheter, can recover the expanded configuration in which the expandable mesh structure forms the spiral shape.

Optionally, the catheter may have an inner diameter of 0.017 inches, 0.021 inches or 0.027 inches.

To achieve the above object, the present invention provides a method of treating an aneurysm. The aneurysm communicates, at a neck thereof, with a blood vessel. The method includes:
placing the aneurysm occlusion device as defined above into the aneurysm;
first releasing the second section of the guide structure that extends out of the expandable mesh structure from the distal end thereof into the aneurysm so that the second section of the guide structure that extends out of the expandable mesh structure from the distal end thereof spirals within the aneurysm into the predetermined shape; and
subsequently releasing the expandable mesh structure into the aneurysm so that, under the guidance of the second section of the guide structure that extends out of the expandable mesh structure from the distal end thereof, the expandable mesh structure continues spiraling within the aneurysm in such a manner that an outer surface of the expandable mesh structure spans over the neck of the aneurysm.

Optionally, the method may further include:
positioning the proximal end of the expandable mesh structure between a wall of the aneurysm and the outer surface in such a manner that the proximal end of the expandable mesh structure is oriented parallel to the wall of the aneurysm without herniating into the blood vessel.

Compared with the prior art, the aneurysm occlusion device, the therapeutic apparatus for aneurysm occlusion and the aneurysm occlusion system of the present invention offer the following advantages:
Firstly, the expandable mesh structure in the aneurysm occlusion device of the present invention has the expanded configuration, in which it assumes the spiral shape spiraling from the distal end to the proximal end thereof, and the collapsed configuration for its delivery into an aneurysm from a blood vessel. With this design, the expandable mesh structure will encounter less friction during its release in the aneurysm's cavity, which enables the expandable mesh structure to more robustly and easily recover the predetermined shape in the aneurysm and more easily achieve the occlusion of the aneurysm neck. Moreover, the spiral shape of the expandable mesh structure is arranged such that the distal end of the device is not oriented toward a wall of the aneurysm and the proximal end of the device is confined between an outer surface of the largest spiral turn in the expandable mesh structure and the wall of the aneurysm so as to be parallel to the wall of the aneurysm (including tangential scenarios). As a result, both the proximal and distal ends of the device will have no impact on the aneurysm wall, avoiding the risk of rupture of the aneurysm. In addition, the outer surface of the largest spiral turn in the expandable mesh structure in the expanded configuration can better cover and block the aneurysm neck, more easily induce thrombosis in the aneurysm, and circumvent the problem that a proximal anchor of the expandable mesh structure is positioned around a center of the aneurysm neck opening or herniate into the parent blood vessel. Thus, endothelialization of the aneurysm neck can be accelerated, and the risk of vascular stenosis can be avoided.

Secondly, during the delivery from the blood vessel into the aneurysm, both the expandable mesh structure and the guide structure can be compressed into linear shapes and loaded in a catheter. This allows the device to have a reduced size for delivery and facilitates its delivery through a catheter with a small inner diameter. In this way, it can be delivered to reach a wider variety of lesions or through narrower blood vessels, making it usable in an expanded spectrum of therapeutic applications. In particular, the expandable mesh structure can be deployed with its spiral shape not having to be oriented in a specific direction and thus can be suitably used to treat both regular and irregular aneurysms. Moreover, it can conform to different aneurysm shapes and can more easily achieve aneurysm occlusion. In addition, the aneurysm occlusion device of the present invention can achieve embolization in one pass, dispensing with the use of multiple embolization devices. Therefore, it enables higher embolization efficiency, simpler operation, less reliance on a physician's surgical experience and reduced surgical complexity and time.

Thirdly, since it is intended to occlude the aneurysm neck opening with the outer surface of the largest spiral turn in the expandable mesh structure, the outer surface of the largest spiral turn is designed to match an inner diameter of the aneurysm. This enables a large contact area, high support strength and stable occlusion by the aneurysm occlusion device without easy displacement. In particular, when the expandable mesh structure has a three-dimensional spiral shape spiraling from the distal end to the proximal end thereof in the expanded configuration, there will be an even larger contact area between the outer diameter of the largest spiral turn and the aneurysm wall, which enables the device to more firmly stay within the aneurysm, additionally reduces the chance of displacement and further lowers the risk of the proximal end of the expandable mesh structure herniating into the parent blood vessel.

Fourthly, the guide structure in the aneurysm occlusion device of the present invention is preferably configured so that the first section is received in the inner cavity of the expandable mesh structure, while the second section extends out of the expandable mesh structure from the distal end thereof and has a spiral shape in the expanded configuration. With this arrangement, the distal spiral-shaped section of the guide structure can better guide the expandable mesh structure to enable it to more easily and better recover the spiral shape in the aneurysm and more easily completely pack the aneurysm. Moreover, the distal spiral-shaped section of the guide structure can reduce resistance to advancement of the expandable mesh structure. The guide structure can relieve tension from the expandable mesh structure during its release, thereby reducing its impact on the aneurysm wall. In particular, when the guide structure is at least partially made of a radiopaque material, this single component combines radiopacity and guidance functions, resulting in increased structural simplicity and dispensing with the need to arrange separate radiopaque components at the proximal and distal ends of the expandable mesh structure or to make the entire expandable mesh structure radiopaque. As a result, the device has improved radiopacity, and the expandable mesh structure is more compliant and thus can be more easily advanced and more easily recover its shape.

Fifthly, the guide structure in the aneurysm occlusion device of the present invention is preferred to be a linear structure, which is elongate, flexible and less invasive to the aneurysm wall, additionally reducing the risk of rupture of the aneurysm. In addition, the expandable mesh structure is preferred to have a cross-sectional area, which increases and then decreases, or repeatedly increases and decreases, from the proximal end to the distal end thereof. This design can facilitate configuration of the expandable mesh structure into a fusiform shape (swollen at the middle and tapering to each end), which makes it easy to compress the device into a smaller size. Moreover, it enables the device to be more flexible and pushed and advanced while experiencing less resistance and exerting a reduced impact on the aneurysm wall. Preferably, the distal end of the expandable mesh structure is fixedly connected with a distal radiopaque ring, which can increase smoothness of the distal end and additionally reduce damage caused to the aneurysm wall.

Sixthly, the expandable mesh structure in the aneurysm occlusion device of the present invention is a mesh structure braided from 48-144 filaments having a diameter of 0.0005-0.002 inches. In this way, denser mesh openings can be formed, which can facilitate embolization of the aneurysm, enable the aneurysm wall to be more uniformly stressed and further reduce the risk of rupture of the aneurysm.

Seventhly, in the aneurysm blocking treatment device of the present invention, the push pod is coupled to the proximal end of the expandable mesh structure and preferred to extend in a direction tangential to a spiral contour of the spiral shape of the expandable mesh structure in the expanded configuration. With this design, the push pod can be manipulated to achieve covering of the aneurysm neck opening by the outer surface of the largest spiral turn in the expandable mesh structure with increased surgical accuracy and improved coverage quality.

### BRIEF DESCRIPTION OF THE DRAWINGS

Fig. 1 depicts a top view of an aneurysm occlusion device according to a preferred embodiment of the present invention, in which an expandable mesh structure has one spiral turn when in an expanded configuration, and a portion of a guide structure (a second section thereof) extends out of the expandable mesh structure from a distal end thereof and assumes a spiral shape also having one spiral turn when in an expanded configuration.
Fig. 2 depicts a perspective view of an aneurysm occlusion device according to a preferred embodiment of the present invention, in which an expandable mesh structure has two spiral turns when in an expanded configuration, and a further portion of a guide structure (a second section thereof) extends out of the expandable mesh structure from a distal end thereof and assumes a spiral shape also having two spiral turns when in an expanded configuration.
Fig. 3a depicts a top view of an aneurysm occlusion device according to a preferred embodiment of the present invention, in which an expandable mesh structure has three spiral turns when in an expanded configuration, and the further portion of a guide structure (a second section thereof) extends out of the expandable mesh structure from a distal end thereof and assumes a spiral shape also having three spiral turns when in an expanded configuration.
Fig. 3b depicts a front view of the aneurysm occlusion device of Fig. 3a.
Fig. 4 shows an aneurysm occlusion device according to a preferred embodiment of the present invention, which has been delivered into an aneurysm but not released there yet.
Fig. 5 shows an aneurysm occlusion device according to a preferred embodiment of the present invention, which has been delivered into and completely released in an aneurysm.
Fig. 6 depicts a partially enlarged view of an aneurysm occlusion device according to a preferred embodiment of the present invention, which is covering an aneurysm neck opening.

In these figures,
10 denotes an aneurysm occlusion device;
11, an expandable mesh structure; 111, a distal end of an expandable mesh structure; 112, a proximal end of an expandable mesh structure; 113, a distal section; 114, a middle section; 115, a proximal section;
12, a guide structure; 121, a distal section of a guide structure; 123, a distal end of a guide structure; 13, a proximal radiopaque ring; 14, a distal radiopaque ring;
20, a push pod; 30, an aneurysm; 40, a microcatheter;
D1, an outer diameter of the largest spiral turn in a guide structure; D2, an outer diameter of the largest spiral turn in an expandable mesh structure; and D3, a maximum outer diameter of an expandable mesh structure.

Throughout the figures, the same reference numbers are used to denote the same or like elements.

### DETAILED DESCRIPTION

Objects, advantages and features of the present invention will become more apparent from the following detailed description when taken in conjunction with the accompanying drawings. Note that the figures are provided in a very simplified form not necessarily drawn to exact scale for the only purpose of facilitating easy and clear description of the disclosed embodiments.

As used herein, the singular forms "a", "an" and "the" include plural referents, unless the context clearly dictates otherwise. As used herein, the term "or" is generally employed in the sense including "and/or", unless the context clearly dictates otherwise. The term "plurality" is generally employed in the sense including "two or more than two", unless the context clearly dictates otherwise. The term "several" is generally employed in the sense including "an indefinite number of', unless the context clearly dictates otherwise. The term "proximal end" generally refers to an end closer to an operator operating a medical device, and the term "distal end" generally refers to an end of the device that enters a human body first, unless the context clearly dictates otherwise.

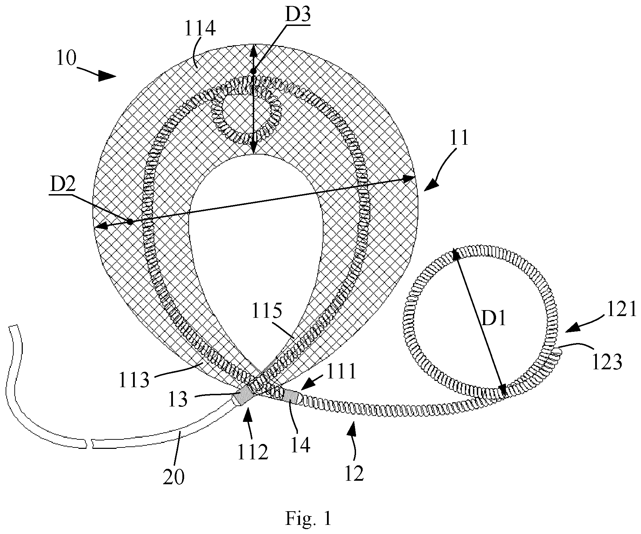

Reference is now made to Fig. 1, which shows an aneurysm occlusion device 10 according to an embodiment of the present invention. The aneurysm occlusion device 10 is used for treatment of aneurysm occlusion. Examples of the aneurysm include, but are not limited to, intracranial aneurysms. Specifically, the aneurysm occlusion device 10 includes an expandable mesh structure 11 and a guide structure 12. At least part of the guide structure 12 is disposed in an inner cavity defined by the expandable mesh structure 11. Preferably, a first section of the guide structure 12 is disposed in the inner cavity of the expandable mesh structure 11, while a second section thereof extends out of the expandable mesh structure 11 from a distal end 111 thereof. In this way, the distal section 121 of the guide structure 12 is exposed out of the expandable mesh structure 11 at the distal end thereof.

The expandable mesh structure 11 has an expanded configuration where it spirals from the distal end 111 to a proximal end 112 thereof and a collapsed configuration for delivery into the aneurysm from a blood vessel. In some embodiments, in the expanded configuration, the expandable mesh structure 11 spirals from the distal end 1 1 1 to the proximal end 112 into a three-dimensional spiral shape. In some other embodiments, in the expanded configuration, the expandable mesh structure 11 spirals from the distal end 111 to the proximal end 112 into a planar spiral shape (i.e., a planar vortex). Additionally, the guide structure 12 is configured so that, when the expandable mesh structure 11 assumes the spiral shape, each of the first section of the guide structure 12 disposed in the inner cavity of the expandable mesh structure 11 and the second section of the guide structure 12 extending out of the expandable mesh structure 11 from the distal end 111 thereof also assumes an expanded configuration where it has a spiral shape. Preferably, the distal section 121 of the guide structure 12 assumes a spiral shape in the expanded configuration. Further, the spiral shape of the distal section 121 of the guide structure 12 may be three-dimensional or planar (i.e., a planar vortex).

When assuming the spiral shape in the expanded configuration, the distal section 121 of the guide structure 12 may spiral either in the same direction as or in a different direction from the expandable mesh structure 11. Preferably, the distal section 121 of the guide structure 12 spirals in the same direction as the expandable mesh structure 11 because this allows good guidance and can prevent bending of the spiral distal section 121 during advancement and delivery, which may affect shape recovery of the expandable mesh structure 11 within the aneurysm. Moreover, an axis about which the distal section 121 of the guide structure 12 spirals may either coincide with an axis about which the expandable mesh structure 11 spirals, or not, without limiting the present invention in any way.

In this embodiment, the expandable mesh structure 11 is configured to assume a three-dimensional spiral shape in the expanded configuration, which enables more secure support, stronger anchoring and reduced likeliness of displacement in the aneurysm. Alternatively, the expandable mesh structure 11 may be configured to be in the shape of a planar vortex in the expanded configuration, which enables the expandable mesh structure 11 to more easily recover and more stably maintain the spiral shape in the aneurysm.

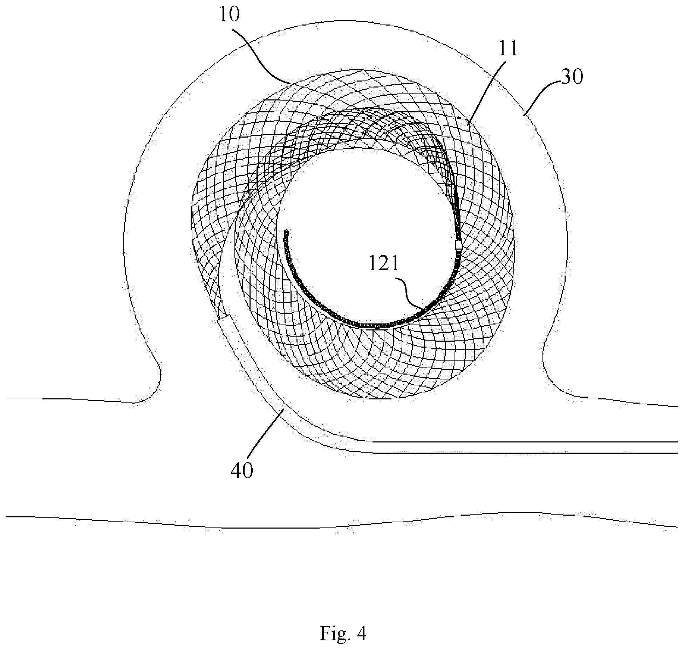

It would be appreciated that the expandable mesh structure 11 and the guide structure 12 may be both resilient structures, which can be compressed by an external force and restore their shape after the external force is removed. More specifically, the expandable mesh structure 11 has a collapsed configuration and an expanded configuration. When loaded within a catheter 40 (see Fig. 4), it assumes the collapsed configuration, in which it may be compressed into a linear shape with a minimized radial size, which facilitates its delivery through the catheter 40 that has a small inner diameter. When released from the catheter 40, the expandable mesh structure 11 can expand by its own resilience into the expanded configuration, in which it recovers a spiral shape. Likewise, the guide structure 12 also has a collapsed configuration and an expanded configuration. When the guide structure 12 is loaded within the catheter 40 together with the expandable mesh structure 11, it assumes the collapsed configuration, in which it is compressed in a similar way as the expandable mesh structure 11, for example, into a linear shape that enables its delivery through the catheter 40. When released from the catheter 40, the guide structure 12 can also expand by its own resilience back into the expanded configuration. In this configuration, the guide structure 12, more preferably, the distal section 121 thereof, assumes a spiral shape.

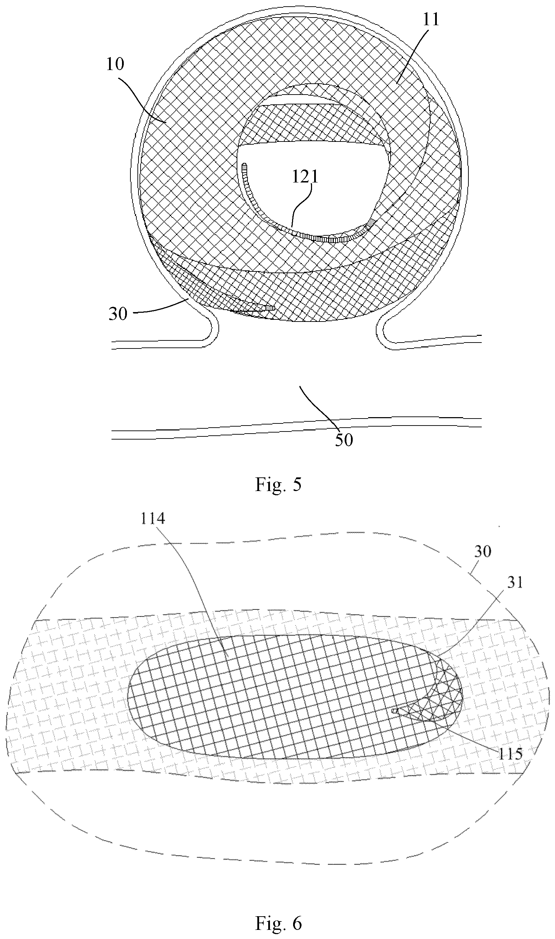

Referring back to Fig. 1, in an embodiment of the present invention, there is also provided a therapeutic apparatus for aneurysm occlusion, including the aneurysm occlusion device 10 and a push pod 20 for advancing the aneurysm occlusion device 10. The proximal end 112 of the expandable mesh structure 11 is configured to be detachably coupled to the push pod 20. Optionally, the proximal end 112 of the expandable mesh structure 11 may be provided with a separate connecting means (not shown) for detachably coupling the push pod 20. Additionally, in the expanded configuration of the expandable mesh structure 11, the push pod 20 is preferred to extend in a direction tangential to a spiral contour of the expandable mesh structure 11. In this way, the expandable mesh structure 11 can be advanced and released so that an outer surface of its largest spiral turn covers an aneurysm neck opening 31 (see Fig. 6). Covering the aneurysm neck opening 31 with the outer surface of the largest spiral turn in the expandable mesh structure 11 can not only ensure sufficient coverage of the aneurysm neck opening 31, but can also avoid the proximal end 112 of the expandable mesh structure 11 from being located around a center of the aneurysm neck opening 31 (e.g., over a central portion thereof) and thus from easily herniating into the parent blood vessel 50 (see Fig. 5). Such herniation may affect the healing of the aneurysm neck and lead to embolism in the blood vessel. Without limiting the prevent invention, the push pod 20 may be detached from the proximal end 112 of the expandable mesh structure 11 thermally, electrically, mechanically, hydrolytically or otherwise as is conventional. The push pod 20 functions primarily to push the aneurysm occlusion device 10 out of the catheter 40 and thereby release it into the aneurysm 30.

The guide structure 12 is preferred to be an elongate structure (i.e., a linear member) in its expanded configuration. For example, it may be a spring coil or a resilient tube formed by cutting. The guide structure 12 has an outer diameter that is much smaller than an outer diameter of the expandable mesh structure 11 in the expanded configuration. In this way, the guide structure 12 is slimmer and more flexible than the expandable mesh structure 11. The guide structure 12 functions primarily to enhance support strength of the expandable mesh structure 11 by the first section disposed in the expandable mesh structure 11 and to guide the expandable mesh structure 11 by the spiral shape of the distal section (i.e., the second section) 121 so as to enable the expandable mesh structure 11 to more easily recover its own spiral shape. Moreover, the distal section 121 can relieve tension from the expandable mesh structure 11 during its release, thereby reducing its impact on the aneurysm wall and resistance to its advancement. Thus, the expandable mesh structure 11 can be more easily pushed and advanced. In particular, since the guide structure 12 is slim and flexible, it will cause less or no damage to the aneurysm wall.

Use of the inventive aneurysm occlusion device 10 will be described below with reference to Figs. 4 to 6. However, the following description is not intended to be construed as limiting the present invention in any way. Rather, it is only intended to explain a preferred embodiment of how to operate the device.

At first, the aneurysm occlusion device 10 is loaded into the catheter 40 for delivery. The aneurysm occlusion device 10 is loaded in the collapsed configuration, in which both the expandable mesh structure 11 and the guide structure 12 are elongated, preferably, into linear shapes, resulting in an overall radial size of the device that is small enough to allow the device to be accommodated in the catheter 40 that has a small inner diameter. Optionally, the inner diameter of the catheter 40 may be 0.017 inches, 0.021 inches or 0.027 inches. After that, as shown in Fig. 4, a distal end of the catheter 40 is held stationary at a proximal end of the aneurysm 30, and the aneurysm occlusion device 10 is then released. In the release process, the push pod 20 (not shown in Fig. 4) may be manipulated to distally push the device 10 or proximally retract the catheter 40 to first release the distal section 121 (i.e., the second section 121) of the guide structure 12, which then recoils in the aneurysm into the intended spiral shape. Since the distal section 121 of the guide structure 12 is a slim flexible spiral structure, it encounters less friction in the aneurysm and thus can easily recover the spiral shape. With the aneurysm occlusion device 10 being further pushed, release of the expandable mesh structure 11 starts. Under the guidance of the spiral shape of the distal section 121 of the guide structure 12, the expandable mesh structure 11 increasingly spirals and its outer diameter continues expanding, until the entire aneurysm occlusion device 10 complete fills and packs the aneurysm, as shown in Fig. 5. At this time, the outer surface of the largest spiral turn in the aneurysm occlusion device 10 covers the aneurysm neck from an inner side thereof, and the whole device stably spirals within the aneurysm 30, achieving stable, compliant packing. Finally, after desired packing quality is confirmed, the push pod 20 may be electrically detached from the proximal end 112 of the expandable mesh structure 11, and the microcatheter 40 and push pod 20 may be withdrawn, ending the process for occluding the aneurysm 30. It is to be noted that, in Figs. 4 to 6, for ease of illustration, the section of the guide structure 12 in the expandable mesh structure 11 is not shown.

As shown in Fig. 5, after the completion of the occlusion process, a distal end of the aneurysm occlusion device 10, especially the distal end of the expandable mesh structure 11, is not oriented toward the aneurysm wall and thus has no impact thereon. Moreover, the proximal end of the expandable mesh structure 11 is confined between the outer surface of the largest spiral turn in the expandable mesh structure 11 and the aneurysm wall so as to be oriented in parallel to the aneurysm wall. Thus, both the proximal and distal ends of the device have no impact on the aneurysm wall. In addition, the only portion contacting the aneurysm wall is a braided dense mesh. This enables the aneurysm wall to be more uniformly stressed and less damaged. In particular, the expandable mesh structure 11 provides multiple barriers, which can desirably block blood flow and facilitate thrombosis in the aneurysm, thus accelerating embolization of the aneurysm. With additional reference to Fig. 6, it is the outer surface of the largest spiral turn in the expandable mesh structure 11 that blocks the aneurysm neck opening 31 by fitting against the inner wall of the aneurysm 30. This enables the device to have good stability. Further, it is unlikely for a proximal end of the device to herniate into the parent blood vessel 50 (see Fig. 5) and affect endothelialization over the aneurysm neck. Therefore, healing of the aneurysm neck opening can be accelerated, and good embolization results can be obtained. In particular, in case of the expandable mesh structure 11 assuming a planar spiral shape in the expanded configuration, the distal end of the device can be completely wrapped within the expandable mesh structure 11. This additionally reduces the chance of the distal end having an impact on the aneurysm wall and even more effectively lowers the risk of rupture of the aneurysm.

Further, at least part of the guide structure 12 is formed of a radiopaque material. More preferably, the first section of the guide structure 12 disposed in the inner cavity of the expandable mesh structure 11 is made of a radiopaque material. The radiopacity of the guide structure 12 under X-ray fluoroscopy enables easy location of the expandable mesh structure 11 and dispenses with the use of a separate radiopaque component, resulting in increased structural simplicity. As a result, the expandable mesh structure 11 is more compliant and can more easily recover the spiral shape in the aneurysm. Further, the guide structure 12 may be overall radiopaque. For example, it may be entirely made of a radiopaque material. The present invention is not limited to any particular radiopaque material of the guide structure 12, and for example, platinum (Pt), platinum-iridium (Pt-Ir) alloys, gold (Au), platinum-tungsten (Pt-W) alloys and the like can be suitably used. In some embodiments, the guide structure 12 may be formed of a non-radiopaque material, such as a nickel-titanium alloy or stainless steel. In a particular embodiment, the guide structure 12 may be obtained by densely winding a metal wire (e.g., a platinum-tungsten alloy, a nickel-titanium alloy, stainless steel or the like) on a metal core bar into a primary coil and then shaping a distal section 121 of the primary coil (which is a linear structure, also called a coil) into the spiral-shaped distal section 121 of the guide structure 12. In another embodiment, the guide structure 12 may be obtained by cutting a metal tube into a flexible mesh tube and stretching the flexible mesh tube into an elongate shape. Preferably, a distal section 121 of the tube is further shaped into the spiral-shaped distal section 121 of the guide structure 12. In yet another embodiment, the guide structure 12 may be a braided structure. For example, filaments may be braided into a tube, which may be then stretched into an elongate shape. Further, a distal section 121 thereof may be further shaped into the spiral-shaped distal section 121 of the guide structure 12. It would be appreciated that, in further embodiments, the fabrication of the guide structure 12 may only involve obtaining an elongate linear structure by braiding, cutting or otherwise but not a stretching process. Furthermore, a distal end 123 of the guide structure 12 is preferably smooth. For example, a light-curable adhesive may be applied to the distal end 123 and then cured there to form a smooth dome-shaped tip, which can reduce damage caused to the aneurysm wall.

The expandable mesh structure 11 is preferably formed of a spirally wound braided tube. Preferably, the braided tube is formed of 48-144 filaments with an outer diameter of 0.0005-0.002 inches. In this way, a dense mesh with a high mesh opening density can be constructed, which can effectively block blood flow and promote thrombosis in the aneurysm and allows better coverage of the aneurysm neck and more uniform stressing of the aneurysm wall, thus additionally reducing the risk of rupture of the aneurysm. Materials suitable for fabrication of the filaments may include shape memory materials, which may be metal materials with shape memory properties, such as nickel-titanium (Ni-Ti) alloys, nickel-titanium-cobalt alloys (Ni-Ti-Co), double-layer composite metal wires (e.g., Ni-Ti@Pt), etc. Suitable materials for the filaments may also include polymer materials with certain shape recovery properties, such as polydioxanone (PDO), poly(l-lactide-co-ε-caprolactone) (PLC), polyurethane (PU), amorphous polynorbornene or combinations thereof. Making the filaments out of a shape memory metal material or a polymer material with certain shape recovery properties can imparts shape memory properties to the braided mesh, which can facilitate the recovery of the original shape. Preferably, the expandable mesh structure 11 is braided from radiopaque filaments. Alternatively, the expandable mesh structure 11 may be braided from both radiopaque and non-radiopaque filaments. With this design, the expandable mesh structure 11 is itself radiopaque under X-ray fluoroscopy, while exhibiting sufficient resilience, which enables the expandable mesh structure 11 to have strong ability to recover and maintain its original shape, thereby dispensing with the use of a separate radiopaque component and increasing compliance of the expandable mesh structure 11. The present invention is not limited to any particular radiopaque material of the radiopaque filaments, and for example, platinum (Pt), platinum iridium alloys (Pt-Ir), gold (Au), platinum-tungsten (Pt-W) alloys and the like can be suitably used. It would be also appreciated that, in case of the guide structure 12 being radiopaque, it is possible for the expandable mesh structure 11 to be either radiopaque or not.

The spiral-shaped distal section 121 of the guide structure 12 in the expanded configuration may have one or more spiral turns. Considering that an excessive outer diameter of the spiral-shaped distal section 121 of the guide structure 12 tends to adversely affect the advancement or expansion of the expandable mesh structure 11, an outer diameter D1 of the largest spiral turn in the spiral-shaped distal section 121 of the guide structure 12 in the expanded configuration is limited to not exceed an outer diameter of a first spiral turn in the expandable mesh structure 11 proximal to the distal end thereof. This condition applies to the case that the distal section 121 of the guide structure 12 has one or more spiral turns. Moreover, when the spiral-shaped distal section 121 of the guide structure 12 in the expanded configuration has a plurality of spiral turns, an outer diameter of a first one of the spiral turns proximal to the distal end of the guide structure 12 is preferably smaller than an outer diameter of any other spiral turn in the guide structure 12, which is preferably not exceed the outer diameter of the first spiral turn in the expandable mesh structure 11 proximal to the distal end thereof. The outer diameters of the other spiral turns in the guide structure 12 may be equal or not, and are preferred to be equal. Here, it would be appreciated that, in case of the distal section 121 of the guide structure 12 having a planar spiral shape, the outer diameters of the other spiral turns in the guide structure 12 are not equal, and in case of the distal section 121 of the guide structure 12 having a three-dimensional spiral shape, the outer diameters of the other spiral turns in the guide structure 12 may be equal or not. Moreover, the outer diameter of the first spiral turn in the guide structure 12 proximal to the distal end thereof is 2/3-3/4 of the outer diameter of any other spiral turn in the guide structure 12, which is preferably not smaller than 2/3 of the outer diameter of the first spiral turn in the expandable mesh structure 11 proximal to the distal end thereof. This design is advantageous in that, during the release and shape recovery of the expandable mesh structure 11, it can provide a space for the packing by the expandable mesh structure 11 and prevent the spiral turns in the distal section 121 of the guide structure 12 from occupying additional space and hence from affecting the release and shape recovery of the expandable mesh structure 11 in the aneurysm. It would be appreciated that the first spiral turn of the guide structure 12 refers to its most distal spiral turn. It would be appreciated that, for a planar spiral shape, an outer diameter of its largest spiral turn is just its maximum outer diameter, while for a three-dimensional spiral shape, an outer diameter of its largest spiral turn is a maximum outer diameter of its axial projection. The largest spiral turn refers to a spiral turn with the largest outer diameter.

The number of spiral turns in the expandable mesh structure 11 may be determined according to the actual size of an aneurysm to be treated. Fewer spiral turns make the expandable mesh structure 11 suitable for use in the treatment of a smaller aneurysm, and more spiral turns enable it to be used in the treatment of a bigger aneurysm. In this embodiment, the spiral-shaped expandable mesh structure 11 in the expanded configuration may have one or more spiral turns, optionally 1-3 spiral turns. Further, considering that when the number of spiral turns is greater than 3, the aneurysm occlusion device 10 will encounter increased resistance to advancement and greater friction during shape recovery in the aneurysm, which may make it difficult for the expandable mesh structure 11 to recover the spiral shape, the spiral-shaped expandable mesh structure 11 in the expanded configuration preferably has no more than 3 spiral turns, more preferably, 1-3 spiral turns.

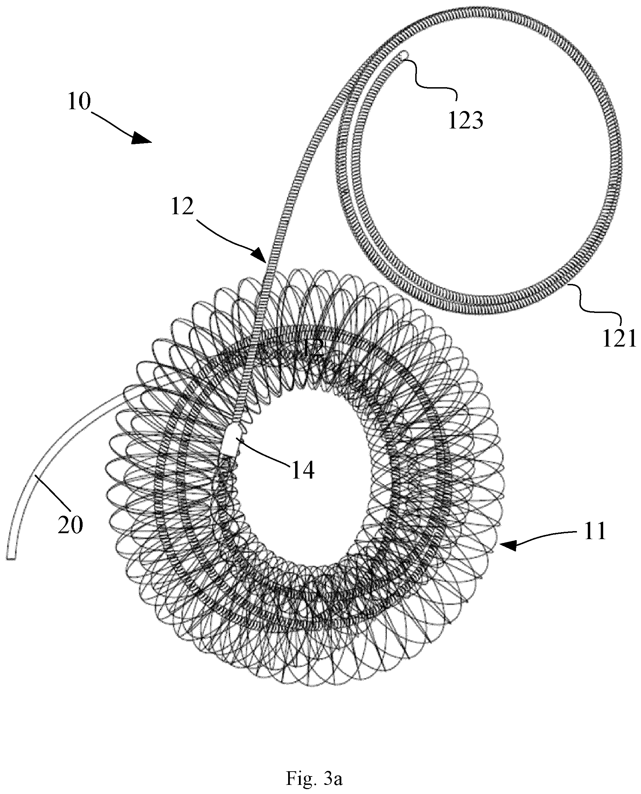

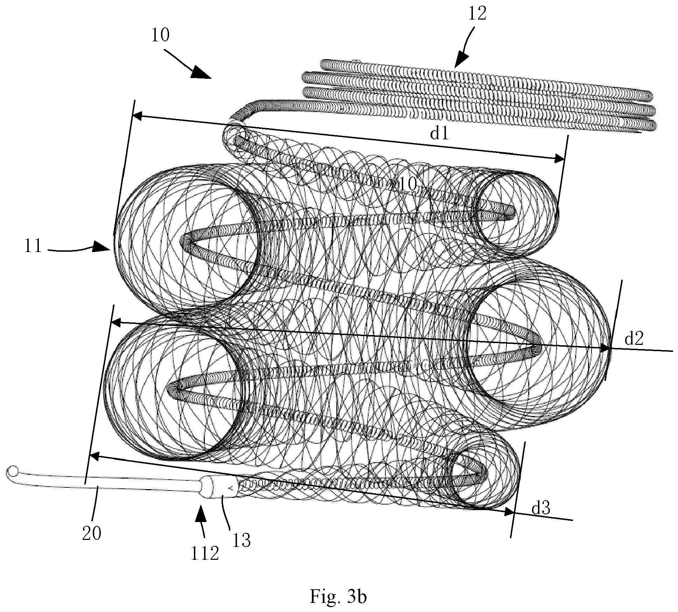

In this embodiment, in case of the spiral-shaped expandable mesh structure 11 in the expanded configuration having a plurality of spiral turns, for example, as shown in Fig. 3b, the outer diameter d1 of the first spiral turn in the expandable mesh structure 11 proximal to the distal end thereof is preferably smaller than an outer diameter d2 of any middle one of the spiral turns in the expandable mesh structure 11. More preferably, an outer diameter d3 of a last one of the spiral turns in the expandable mesh structure 11 proximal to the proximal end thereof is also smaller than the outer diameter d2 of any middle spiral turn in the expandable mesh structure 11. It would be appreciated that a middle spiral turn refers to another spiral turn than the first spiral turn proximal to the distal end and the last spiral turn proximal to the proximal end. This design is advantageous in facilitating shape recovery of the expandable mesh structure 11 by minimizing resistance, and in providing sufficient support, which can further stabilize the aneurysm occlusion device 10 and enables it to completely pack the interior of the aneurysm with an increased packing density. Preferably, the outer diameter d1 of the first spiral turn in the expandable mesh structure 11 proximal to the distal end thereof is equal to the outer diameter d3 of the last spiral turn proximal to the proximal end, as shown in Figs. 3a and 3b. Moreover, the outer diameter d1 of the first spiral turn in the expandable mesh structure 11 proximal to the distal end thereof may be 2/3 of the outer diameter d2 of any middle spiral turn in the expandable mesh structure 11. In addition, when the expandable mesh structure 11 has a plurality of middle spiral turns, these middle spiral turns are preferred to have equal outer diameters d2. It would be appreciated that the first spiral turn of the expandable mesh structure 11 refers to its most distal spiral turn.

For example, as shown in Fig. 1, the spiral-shaped distal section 121 of the guide structure 12 in the expanded configuration may have one spiral turn, and the spiral-shaped expandable mesh structure 11 in the expanded configuration may also have one spiral turn. In this case, preferably, the proximal end 112 and the distal end 111 of the expandable mesh structure 11 are located at the same radial position on the same side of an axis of spiral thereof. Moreover, the expandable mesh structure 11 spirals in the same direction as the distal section 121 of the guide structure 12. More preferably, the outer diameter D1 of the largest spiral turn in the guide structure 12 is 2/3 of an outer diameter D2 of the largest spiral turn in the expandable mesh structure 11. Additionally, the maximum outer diameter D3 of the expandable mesh structure 11 is preferably 1/2 of the outer diameter D2 of the largest spiral turn. In this example, the guide structure 12 can guide shape recovery of the expandable mesh structure 11 within the aneurysm through the single spiral turn of the distal section 121 and reduce damage to the aneurysm wall caused by the distal end of the expandable mesh structure 11.

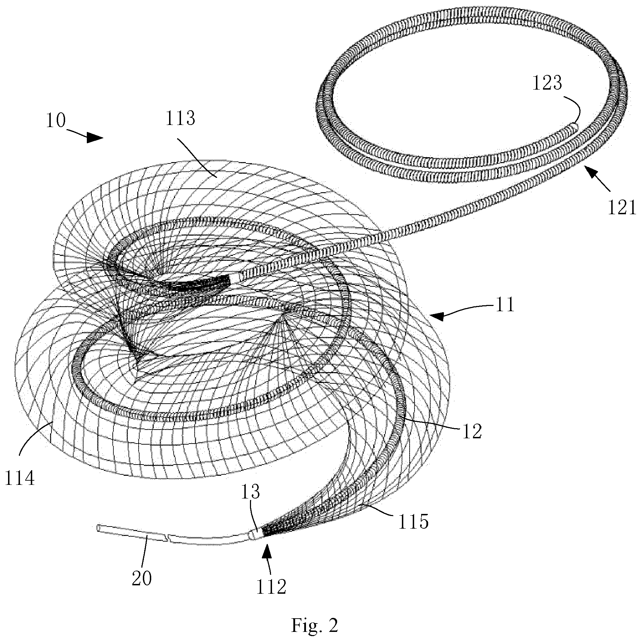

Alternatively, as shown in Fig. 2, the spiral-shaped distal section 121 of the guide structure 12 in the expanded configuration may have two spiral turns, and the spiral-shaped expandable mesh structure 11 in the expanded configuration may also have two spiral turns. In this case, the outer diameter of first spiral turn in the guide structure 12 proximal to the distal end thereof is preferably 2/3 of the outer diameter of the second spiral turn, which is preferably equal to the outer diameter d1 of the first spiral turn in the expandable mesh structure 11 proximal to the distal end thereof. Moreover, the outer diameter d1 of the first spiral turn in the expandable mesh structure 11 proximal to the distal end thereof is preferably 3/4 of the outer diameter d2 of the second (largest) spiral turn therein. In addition, the maximum outer diameter D3 of the expandable mesh structure 11 is preferably 1/3 of the outer diameter D2 of its largest spiral turn. Compared with the case of a single spiral turn, the expandable mesh structure 11 with two spiral turns is able to more completely pack the aneurysm, enables better coverage of the aneurysm neck opening by the outer surface of the largest spiral turn and can be used in the treatment of a bigger aneurysm.

Still alternatively, as shown in Figs. 3a and 3b, the spiral-shaped distal section 121 of the guide structure 12 in the expanded configuration may have three spiral turns, and the spiral-shaped expandable mesh structure 11 in the expanded configuration may also have three spiral turns. In this case, the outer diameter of the first spiral turn in the guide structure 12 proximal to the distal end thereof is preferably 2/3 of the outer diameter of the second spiral turn, which is equal to the outer diameter of the third spiral turn. More preferably, the outer diameter of the third spiral turn in the guide structure 12 is equal to the outer diameter d1 of the first spiral turn in the expandable mesh structure 11 proximal to the distal end thereof, which is preferably 2/3 of the outer diameter d2 of the second spiral turn. The outer diameter d3 of the third spiral turn in the expandable mesh structure 11 is preferred to be 2/3 of the outer diameter d2 of the second spiral turn. That is, the second spiral turn in the expandable mesh structure 11 has the largest outer diameter d2. Further, the maximum outer diameter D3 of the expandable mesh structure 11 is preferably 1/4 of the outer diameter D2 of the largest spiral turn. In this case, since the expandable mesh structure 11 has more spiral turns, it is able to even more completely pack the aneurysm, enables even better coverage of the aneurysm neck opening by the outer surface of at least one spiral turn and can be used in the treatment of an even bigger aneurysm.

It would be appreciated that, in the expanded configuration, the present invention is not limited to the above examples because the expandable mesh structure 11 may include even more spiral turns. In addition, the number of spiral turns in the expandable mesh structure 11 may be equal to the number of spiral turns in the distal section 121 of the guide structure 12 or not, without departing from the scope of the present invention. It would be appreciated that the expandable mesh structure 11 with more spiral turns can even more completely pack the aneurysm and be used in the treatment of an even bigger aneurysm.

Further, in case of the spiral-shaped distal section 121 of the guide structure 12 in the expanded configuration having a plurality of spiral turns, the outer diameter of the first spiral turn of the guide structure 12 proximal to the distal end thereof is slightly smaller than, preferably 2/3-3/4 of, the outer diameter of any other spiral turn in the guide structure 12. The outer diameter of any other spiral turn in the guide structure 12 than the first spiral turn is preferred to be not smaller than 2/3 of, but not exceed, the outer diameter of the first spiral turn in the expandable mesh structure 11 proximal to the distal end thereof.

Further, in the expanded configuration, the maximum outer diameter D3 of the expandable mesh structure 11 is preferably not smaller than 1/4, more preferably 1/3-1/2, of the outer diameter D2 of the largest spiral turn in the expandable mesh structure 11. This is advantageous in ensuring sufficient friction between the outer surface of the largest spiral turn in the expandable mesh structure 11 and the aneurysm wall, which prevents easy displacement of the aneurysm occlusion device 10 and disperses the support from the aneurysm occlusion device 10 to avoid the aneurysm wall from being damaged due to excessive local pressure. Additionally, it can be ensured that the outer surface of the largest spiral turn in the expandable mesh structure 11 covers the aneurysm neck as much as possible to accelerate thrombosis in the aneurysm. In this embodiment, the outer diameter D2 of the largest spiral turn in the expandable mesh structure 11 is determined principally according to the size of the aneurysm to be treated. For example, the maximum outer diameter D3 of the expandable mesh structure 11 may be 2.0-8.0 mm, and the outer diameter D2 of the largest spiral turn therein may be 3.0-25 mm.

Further, in order to facilitate advancement of the expandable mesh structure 11, the proximal end 112 and the distal end 111 of the expandable mesh structure 11 are preferably both secured to the guide structure 12. Preferably, an axial length of the first section of the guide structure 12 disposed in the inner cavity of the expandable mesh structure 11 in the expanded configuration is not smaller than an axial length of the expandable mesh structure 11 in the collapsed configuration. This ensures that the expandable mesh structure 11 can be successfully compressed and can successfully expand without being affected by the guide structure 12.

Additionally, a proximal radiopaque ring 13 may be attached to the proximal end 112 of the expandable mesh structure 11. On one aspect, the radiopacity of the proximal radiopaque ring 13 under X-ray fluoroscopy allows location of the proximal end 112 of the expandable mesh structure 11 and increases overall radiopacity of the entire device. On another aspect, end portions of filaments in the expandable mesh structure 11 can be hidden within the proximal radiopaque ring 13 and thus cause less damage to the aneurysm wall. Further, a distal radiopaque ring 14 may be attached to the distal end 111 of the expandable mesh structure 11 (see Fig. 1). On one aspect, the radiopacity of the distal radiopaque ring 14 under X-ray fluoroscopy allows location of the distal end 111 of the expandable mesh structure 11 and increases the overall radiopacity of the entire device. On another aspect, end portions of filaments in the expandable mesh structure 11 can be hidden within the distal radiopaque ring 14 and thus cause less damage to the aneurysm wall. Optionally, the proximal radiopaque ring 13 may be attached with an adhesive to both the proximal end 112 of the expandable mesh structure 11 and the proximal end 122 of the guide structure 12. Similarly, the distal radiopaque ring 14 may be attached with an adhesive to both the distal end 111 of the expandable mesh structure 11 and the guide structure 12. It would be appreciated that, when the expandable mesh structure 11 itself, or the guide structure 12, is radiopaque, the proximal radiopaque ring 13 and/or the distal radiopaque ring 14 may be either provided or not.

In this embodiment, a cross-sectional area of the expandable mesh structure 11 is preferred to increase and then decrease from the proximal end 112 to the distal end 111, as particularly shown in Figs. 1 and 2. In this case, the outer diameter of the expandable mesh structure 11 is not constant. Additionally, the expandable mesh structure 11 includes, sequentially joined side by side axially, a distal section 113, a middle section 114 and a proximal section 115. The distal section 113 preferably has a cross-sectional area (or outer diameter) increasing from the distal end 111 to the middle section 114, and/or the proximal section 115 preferably has a cross-sectional area (or outer diameter) increasing from the proximal end 112 to the middle section 114. In other embodiments, the cross-sectional area of the expandable mesh structure 11 may repeatedly increase and decrease from the proximal end 112 to the distal end 111. That is, it may repeatedly widen and narrow. This design can facilitate configuration of the expandable mesh structure 11 into a fusiform shape (swollen at the middle and tapering to each end), which makes it easy to be compressed into a smaller size. Moreover, it enables the aneurysm occlusion device to be more flexible and pushed and advanced while experiencing less resistance and exerting a reduced impact on the aneurysm wall. The distal section 113 of the expandable mesh structure 11 can function as a guide means, which facilitates shape recovery of the expandable mesh structure 11. The expandable mesh structure 11 may have a greater mesh opening density at both the distal section 113 and the proximal section 115. In particular, a greater mesh opening density of the distal section 113 enables the aneurysm occlusion device 10 to have increased strength, better support stability within the spiral shape and higher resistance to displacement. The present invention is not limited to any particular mesh opening density distribution of the middle section 114 of the expandable mesh structure 11, and for example, the middle section 114 may have a uniform or non-uniform mesh opening density.

It would be appreciated that, the distal section 121 of the guide structure 12, as well as the expandable mesh structure 11, may alternatively assume the shape of a planar spiral. In this case, the distal section 121 of the guide structure 12 may spiral in the same direction as the expandable mesh structure 11, preferably within the same plane. In addition, in case of the expandable mesh structure 11 being in the shape of a planar spiral, it may define a bulge at the proximal end 112, which can increase friction between the expandable mesh structure 11 and the aneurysm wall, enhance stability of the device, reduce the risk of herniation of the proximal section 115, lower the risk of vascular embolism, improve coverage of the aneurysm neck and accelerate thrombosis in the aneurysm. The bulge may be implemented as a circumferentially continuous closed flange. That is, the bulge may be made up of one circumferentially continuous monolithic ring. Alternatively, the bulge may be implemented as a circumferentially non-continuous closed flange. That is, it may be made up of multiple sectorial projections, which are spaced apart circumferentially. Still alternatively, the bulge may be implemented as an unclosed ring extending circumferentially around the proximal section 115, such as for example, a 1/4 ring, a half ring or a 3/4 ring. Further, in case of the distal section 121 of the guide structure 12 being also in the shape of a planar spiral, the outer diameter of the largest spiral turn in the guide structure 12 is preferred not to exceed the outer diameter of the first spiral turn in the expandable mesh structure 11 proximal to the distal end thereof, in order to avoid affecting advancement and shape recovery of the expandable mesh structure 11. Preferably, in order to enhance overall strength of the entire device, the outer diameter of the largest spiral turn in the guide structure 12 is not smaller than 2/3 of the outer diameter of the first spiral turn in the expandable mesh structure 11 proximal to the distal end thereof. Further, in order for better shape recovery and easier size control to be achieved, adjacent spiral turns in the expandable mesh structure 11 in the shape of a planar vortex are preferably brought in contact with each other.

In embodiments of the present invention, there is also provided an aneurysm occlusion system including the aneurysm occlusion device 10 and the catheter 40. The expandable mesh structure 11 is compressed within the catheter 40 and, after being released from the catheter 40, recovers the expanded configuration in which it assumes the spiral shape.

At last, it is to be noted that, although a few preferred embodiments of the present invention have been described above, the scope of the invention is in no way limited to these embodiments disclosed hereinabove. For example, the present invention is not limited to any particular number of spiral turns in the expandable mesh structure 11 in the expanded configuration, or any particular number of spiral turns in the guide structure 12 in the expanded configuration, or any particular outer diameter of the largest spiral turn in the expandable mesh structure 11 in the expanded configuration, or any particular length of the guide structure 12 within the expandable mesh structure 11. It is to be also noted that, in other embodiments, the guide structure 12 may be entirely disposed in the inner cavity of the expandable mesh structure 11.

Thus, embodiments of the present invention provide an aneurysm occlusion device capable of easier blocking of the neck of an aneurysm while not exerting any impact on a wall of the aneurysm at its distal or proximal end, thereby avoiding the risk of rupture of the aneurysm, increasing coverage of the aneurysm neck and facilitating thrombosis in the aneurysm. Additionally, the present invention can circumvent the problem that a proximal anchor of the expandable structure is located around a center of an opening of the aneurysm neck and may herniate into the parent blood vessel. In this way, endothelialization of the aneurysm neck can be accelerated, and the risk of vascular stenosis can be avoided.

The description presented above is merely that of a few preferred embodiments of the present invention and does not limit the scope thereof in any sense. Any and all changes and modifications made by those of ordinary skill in the art based on the above teachings fall within the scope as defined in the appended claims.

## Claims

1. An aneurysm occlusion device, comprising:
an expandable mesh structure having an expanded configuration, in which the expandable mesh structure forms a spiral shape by spiraling from a distal end to a proximal end thereof, and a collapsed configuration for delivery into an aneurysm from a blood vessel; and
a guide structure, at least a portion of which is disposed in an inner cavity defined by the expandable mesh structure and, when the expandable mesh structure is in the spiral shape, expands into a spiral shape in the inner cavity of the expandable mesh structure.

2. The aneurysm occlusion device according to claim 1, wherein the spiral shape of the expandable mesh structure in the expanded configuration thereof is a three-dimensional spiral shape formed by spiraling from the distal end to the proximal end.

3. The aneurysm occlusion device according to claim 1, wherein the guide structure is at least partially made of a radiopaque material.

4. The aneurysm occlusion device according to any one of claims 1 to 3, wherein the guide structure has a first section that is disposed in the inner cavity of the expandable mesh structure and a second section that extends out of the expandable mesh structure from the distal end thereof.

5. The aneurysm occlusion device according to claim 4, wherein the second section of the guide structure that extends out of the expandable mesh structure from the distal end thereof has a spiral shape when in the expanded configuration.

6. The aneurysm occlusion device according to claim 5, wherein the second section of the guide structure that extends out of the expandable mesh structure from the distal end thereof has a three-dimensional spiral shape.

7. The aneurysm occlusion device according to claim 1, wherein the guide structure has a linear structure.

8. The aneurysm occlusion device according to any one of claims 1 to 3, wherein both the proximal and distal ends of the expandable mesh structure are fixedly connected to the guide structure, and an axial length of a first section of the guide structure that is disposed in the inner cavity of the expandable mesh structure in the expanded configuration is not smaller than an axial length of the expandable mesh structure in the collapsed configuration.

9. The aneurysm occlusion device according to any one of claims 1 to 3, wherein a distal radiopaque ring is fixedly connected to the distal end of the expandable mesh structure, and/or a proximal radiopaque ring is fixedly connected to the proximal end of the expandable mesh structure.

10. The aneurysm occlusion device according to any one of claims 1 to 3, wherein a cross-sectional area of the expandable mesh structure increases and then decreases from the proximal end to the distal end.

11. The aneurysm occlusion device according to claim 10, wherein the expandable mesh structure comprises a proximal section, a middle section and a distal section which are connected axially in sequence, wherein a cross-sectional area of the proximal section gradually increases from a proximal end to a distal end thereof, and/or a cross-sectional area of the distal section gradually increases from a distal end to a proximal end thereof.

12. The aneurysm occlusion device according to claim 10, wherein the cross-sectional area of the expandable mesh structure repeatedly increases and decreases from the proximal end to the distal end.

13. The aneurysm occlusion device according to any one of claims 1 to 3, wherein in the expanded configuration, a maximum outer diameter of the expandable mesh structure is not smaller than 1/4 of an outer diameter of a largest spiral turn in the expandable mesh structure.

14. The aneurysm occlusion device according to claim 13, wherein in the expanded configuration, the maximum outer diameter of the expandable mesh structure is 1/3 to 1/2 of the outer diameter of the largest spiral turn in the expandable mesh structure.

15. The aneurysm occlusion device according to any of claims 1 to 3, wherein the expandable mesh structure is formed by braiding filaments made of a material comprising a shape memory material.

16. The aneurysm occlusion device according to claim 15, wherein the expandable mesh structure is formed by braiding 48-144 filaments having an outer diameter of 0.0005-0.002 inches.

17. The aneurysm occlusion device according to claim 15, wherein the expandable mesh structure is formed by braiding radiopaque filaments, or the expandable mesh structure is formed by braiding both radiopaque and non-radiopaque filaments.

18. The aneurysm occlusion device according to any one of claims 1 to 3, wherein the spiral shape of the expandable mesh structure in the expanded configuration has one spiral turn or a plurality of spiral turns,
wherein in case of the plurality of spiral turns, a first one of the spiral turns in the expandable mesh structure proximal to the distal end thereof has an outer diameter smaller than an outer diameter of each middle one of the spiral turns.

19. The aneurysm occlusion device according to claim 18, wherein in case of the plurality of spiral turns, a last one of the spiral turns in the expandable mesh structure proximal to the proximal end thereof has an outer diameter smaller than the outer diameter of each middle spiral turn.

20. The aneurysm occlusion device according to claim 18, wherein in the spiral shape of the expandable mesh structure in the expanded configuration, the outer diameter of the first spiral turn proximal to the distal end is equal to the outer diameter of the last spiral turn proximal to the proximal end.

21. The aneurysm occlusion device according to claim 18, wherein in the spiral shape of the expandable mesh structure in the expanded configuration, the outer diameter of the first spiral turn proximal to the distal end is 2/3 of the outer diameter of each middle spiral turn.

22. The aneurysm occlusion device according claim 5 or 6, wherein the spiral shape of the second section of the guide structure that extends out of the expandable mesh structure from the distal end thereof in the expanded configuration has one spiral turn or a plurality of spiral turns,
wherein in case of the plurality of spiral turns, a first one of the spiral turns in the guide structure proximal to an distal end thereof has an outer diameter smaller than an outer diameter of each spiral turn other than the first spiral turn.

23. The aneurysm occlusion device according to claim 22, wherein in case of the spiral shape of the second section of the guide structure that extends out of the expandable mesh structure from the distal end thereof in the expanded configuration having the plurality of spiral turns, the outer diameter of each spiral turn in the guide structure does not exceed an outer diameter of a first spiral turn in the expandable mesh structure proximal to the distal end thereof.

24. The aneurysm occlusion device according to claim 23, wherein the outer diameter of the first spiral turn in the guide structure proximal to the distal end thereof is 2/3-3/4 of the outer diameter(s) of the other spiral turn(s), which is/are equal and not smaller than 2/3 of an outer diameter of a first spiral turn in the expandable mesh structure proximal to the distal end thereof.

25. A therapeutic apparatus for aneurysm occlusion, comprising the aneurysm occlusion device according to any one of claims 1 to 24 and a push pod connected to the proximal end of the expandable mesh structure in the aneurysm occlusion device.

26. The therapeutic apparatus for aneurysm occlusion according to claim 25, wherein the push pod extends in a direction tangential to a spiral contour of the spiral shape of the expandable mesh structure in the expanded configuration.

27. An aneurysm occlusion system, comprising the aneurysm occlusion device according to any one of claims 1 to 24 and a catheter, wherein the expandable mesh structure is compressed in the catheter and, after being released from the catheter, can recover the expanded configuration in which the expandable mesh structure forms the spiral shape.

28. The aneurysm occlusion system according to claim 27, wherein the catheter has an inner diameter of 0.017 inches, 0.021 inches or 0.027 inches.
